# EUROPEAN PATENT APPLICATION

(11) **EP 3 290 516 A1**
(43) Date of publication of application: **07.03.2018**
(21) Application number: 17196863.9
(22) Date of filing: 10.02.2005
(51) Int. Cl.: C12N 15/00, C12N 15/09, C12N 15/63

(54) **RECOMBINANT DNA FOR GENE SUPPRESSION**

(30) Priority: 10.02.2004 US 543157 P; 10.02.2004 US 543187 P; 11.08.2004 US 600859 P
(62) Divisional of application: 11160073.0
(71) Applicant: Monsanto Technology LLC, Saint Louis, MO 63167 (US)
(72) Inventor: Malvar, Thomas M., North Stonington, CT Connecticut 06359 (US); Huang, Shihshieh, St. Louis, MO Missouri 63167 (US); Luethy, Michael H., St. Louis, MO Missouri 63167 (US)
(74) Representative: dompatent von Kreisler Selting Werner - Partnerschaft von Patent- und Rechtsanwälten mbB

(57) **Abstract**

Anti-sense-oriented RNA gene suppression agents in the form of a loop of anti-sense-oriented RNA is produced in cells of transgenic organisms, e.g. plants, by transcription from a recombinant DNA construct which comprises in 5' to 3' order a promoter element operably linked to an anti-sense-oriented DNA element and a complementary DNA element.

## Description

### Cross Reference to Related Applications

This application claims priority under 35 U.S.C. 119(e) to provisional applications Serial No. 60/543,157, filed February 10, 2004, No. 60/543,187, filed February 10, 2004 and No. 60/600,859, filed August 11, 2004, the disclosures of all of which are incorporated herein by reference in their entireties.

### Incorporation of Sequence Listing

A computer readable form of the sequence listing is contained in the file named "53428B.ST25.txt" which is 21 kb (measured in MS-Windows) and was created on February 9, 2005 and is located on a CDROM, which is filed herewith and herein incorporated by reference.

### Field of the Invention

Disclosed herein are seeds for transgenic corn having elevated amino acid level, recombinant DNA constructs for producing gene-suppressing loops of anti-sense RNA and methods of making and using such constructs and transgenic plants expressing gene-suppressing loops of anti-sense RNA.

### Background

Certain plants have low levels of specific amino acids compared to other plants, e.g. corn has low levels of lysine, methionine and tryptophan. Efforts to increase amino acid levels in transgenic plants include expressing recombinant DNA which encodes proteins in an amino acid synthesis pathway at higher levels than native genes. One such gene for producing enhanced levels of lysine in corn is a bacterial dihydropicolinic acid synthase. A concept for even more enhanced levels of amino acids includes suppression of genes encoding proteins in amino acid catabolic pathways.

Gene suppression includes any of the well-known methods for suppressing transcription of a gene or the accumulation of the mRNA corresponding to that gene thereby preventing translation of the transcript into protein. More particularly, gene suppression mediated by inserting a recombinant DNA construct with anti-sense oriented DNA to regulate gene expression in plant cells is disclosed in U.S. Patent 5,107,065 (Shewmaker et al.) and US Patent 5,759,829 (Shewmaker et al.)*.* Plants transformed using such anti-sense oriented DNA constructs for gene suppression can comprise integrated DNA arranged as an inverted repeat that resulted from co-insertion of several copies of the transfer DNA (T-DNA) into plants by *Agrobacterium-mediated* transformation, as disclosed by Redenbaugh et al. in "Safety Assessment of Genetically Engineered Flavr Savr™ Tomato, CRC Press, Inc. (1992). Inverted repeat insertions can comprise a part or all of the T-DNA, e.g. contain an inverted repeat of a complete or partial anti-sense construct. Screening for inserted DNA comprising inverted repeat elements can improve the efficiency of identifying transformation events effective for gene silencing when the transformation construct is a simple anti-sense DNA construct.

Gene suppression triggered by inserting a recombinant DNA construct with sense-oriented DNA to regulate gene expression in plants is disclosed in U.S. Patent 5,283,184 (Jorgensen et al.) and U.S. Patent 5,231,020 (Jorgensen et al.)*.* Inserted T-DNA providing gene suppression in plants transformed with such sense constructs by *Agrobacterium* is organized predominately in inverted repeat structures, as disclosed by Jorgensen et al., Mol. Gen. Genet., 207: 471-477 (1987). See also Stam et al., The Plant Journal, 12: 63-82 (1997) and De Buck et al., Plant Mol. Biol. 46 433-445 (2001), who used segregation studies to support Jorgensen's finding that in many events gene silencing is mediated by multimeric transgene T-DNA where the T-DNAs are arranged in inverted repeats. Screening for inserted DNA comprising inverted repeat elements can improve the gene silencing efficiency when transforming with simple sense-orientated DNA constructs.

Gene silencing can also be effected by transcribing RNA from both a sense and an anti-sense oriented DNA using two separate transcription units, e.g. as disclosed by Shewmaker et al. in U.S. Patent 5,107,065 where in Example 1 a binary vector was prepared with both sense and anti-sense *aroA* genes. Similar constructs are disclosed in International Publication No. WO 99/53050 (Waterhouse et al.). See also U.S. Patent 6,326,193 where gene targeted DNA is operably linked to opposing promoters.

Gene suppression can be achieved in plants by providing transformation constructs that are capable of generating an RNA that can form double-stranded RNA along at least part of its length. Gene suppression in plants is disclosed in EP 0426195 A1 (Goldbach et al.) where recombinant DNA constructs for transcription into hairpin RNA provided transgenic plants with resistance to tobacco spotted wilt virus. See also Sijen et al., The Plant Cell, Vol. 8, 2277-2294 (1996) which discloses the use of constructs carrying inverted repeats (sense followed by anti-sense) of a cowpea mosaic virus gene in transgenic plants to mediate virus resistance. See also International Publication No. 98/53083 (Grierson et al.) and related U.S. Patent Application Publication No. 2003/0175965 A1 (Lowe et al.) which disclose gene suppression, using a double stranded RNA construct comprising a gene coding sequence preceded by an inverted repeat of 5'UTR. Constructs for posttranscriptional gene suppression in plants by double-stranded RNA of the target gene are also disclosed in International Publication No. WO 99/53050 (Waterhouse et al.) and International Publication No. WO 99/49029 (Graham et al.)*.* See also U.S. Patent Application Publication No. 2002/0048814 A1 (Oeller) where DNA constructs are transcribed to sense or anti-sense RNA with a hairpin-forming poly(T)-poly(A) tail. See also U.S. Patent Application Publication No. 2003/0018993 A1 (Gutterson et al.) where sense or anti-sense DNA is followed by an inverted repeat of the 3' untranslated region of the NOS gene. See also U.S. Patent Application Publication No. 2003/0036197 A1 (Glassman et al.) where RNA for reducing the expression of target mRNA comprises a part with homology to target mRNA and a part with complementary RNA regions that are unrelated to endogenous RNA.

The production of dsRNA in plants to inhibit gene expression, e.g. in a nematode feeding on the plant, is disclosed U.S. Patent 6,506,559 (Fire et al.)*.* Multi-gene suppression vectors for use in plants are disclosed in U.S. Patent Application No. 10/465,800 (Fillatti).

Transcriptional suppression such as promoter *trans* suppression can be affected by a expressing a DNA construct comprising a promoter operably linked to inverted repeats of promoter DNA from a target gene. Constructs useful for such gene suppression mediated by promoter *trans* suppression are disclosed by Mette et al., The EMBO Journal, Vol. 18, pp. 241-148, (1999) and by Mette et al., The EMBO Journal, Vol. 19, pp. 5194-5201-148, (2000), both of which are incorporated herein by reference.

All of the above-described patents, applications and international publications disclosing materials and methods for gene suppression in plants are incorporated herein by reference.

### Summary of the Invention

This invention provides methods and recombinant DNA constructs useful for producing anti-sense-oriented RNA for gene suppression in transgenic organisms. In one aspect of the invention recombinant DNA constructs comprise in 5' to 3' order a promoter element operably linked to an anti-sense-oriented DNA element and a sense-oriented DNA element, where the sense-oriented DNA element is shorter than the anti-sense-oriented DNA element, and sense-oriented RNA transcribed by the sense-oriented DNA is complementary to the 5'-most end of anti-sense-oriented RNA transcribed by the anti-sense-oriented DNA element, wherein said transcribed RNA forms a into a loop of anti-sense-oriented RNA for suppressing said at least one target gene.

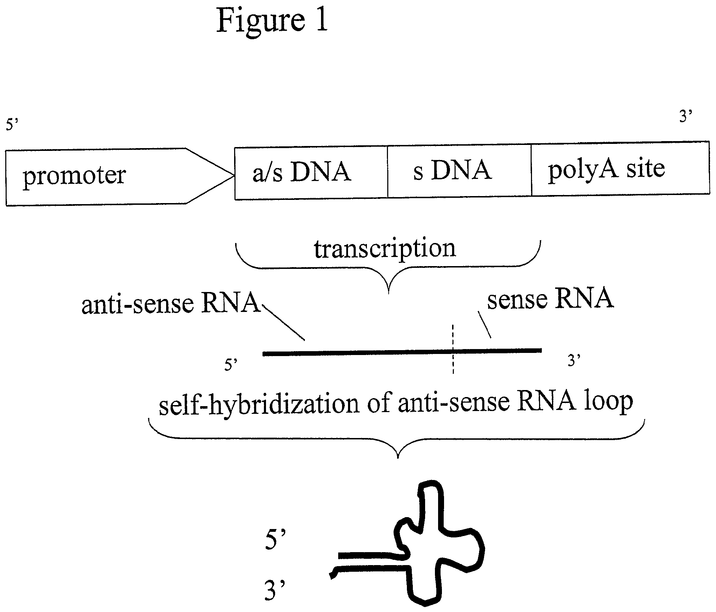

The sense-oriented DNA can be cloned as an inverted repeat of 5'-most segment of the anti-sense-oriented DNA element. Constructs with such sense-oriented DNA are transcribed to RNA that forms a loop of anti-sense-oriented RNA closed at its ends with a double-stranded RNA (dsRNA) segment, e.g. as illustrated in Figure 1. To form an anti-sense-oriented RNA loop the complementary DNA element is conveniently not more than about one-half the length of the anti-sense-oriented DNA element, often not more than one-third the length of said anti-sense-oriented DNA element, e.g. not more than one-quarter the length of said anti-sense-oriented DNA element. The overall lengths of the combined DNA elements can vary. For instance, the anti-sense-oriented DNA element can consist of from 500 to 5000 nucleotides and the complementary DNA element can consist of from 50 to 500 nucleotides. In many cases it is useful for the anti-sense-oriented DNA segment to be more than twice the length of the sense-oriented DNA segment to allow for formation of an anti-sense-oriented RNA loop.

The anti-sense transcription unit can be designed to suppress multiple genes where the DNA is arranged with two or more anti-sense-oriented elements from different genes targeted for suppression followed by a complementary sense-oriented element, e.g. complementary to at least a part of the 5'most anti-sense element.

Aspects of this invention provide methods of suppressing the expression of a gene by providing in the cells of a plant a gene-suppressing, recombinant DNA construct of this invention that transcribes to an anti-sense loop of RNA. In other aspects of the invention, e.g. for providing traits other than plants with enhanced amino acid, the gene targeted for suppression can be a plant gene, a plant pest gene or a plant pathogen gene or a combination thereof. In constructs, methods and plants of this invention the gene targeted for silencing can be a native gene or an exogenous gene or a gene in an organism that ingests or contacts plant tissue including cells comprising anti-sense RNA in a loop. Plant pathogens include viruses such a cucumber mosaic virus; plant pests include nematodes such as soybean cyst nematode and root knot nematode, insect larvae such a *lepidopteran larvae,* sucking insects such as aphids and leaf eating insects such as locust.

### Brief Description of the Drawing

Figure 1 is a schematic illustration of a recombinant DNA construct useful in this invention to produce an anti-sense-oriented loop of RNA.
Figure 2 is a Western analysis indicating gene suppression using a construct of this invention.
Figure 3 shows mass spectroscopy spectra indicating zein content in seeds.

### Detailed Description

SEQ ID NO:1 and SEQ ID NO:2 are nucleotide sequences of recombinant DNA constructs useful for transcribing RNA that can form an anti-sense-oriented RNA loop for suppressing one or multiple genes in transgenic plants. See Tables 1 and 2 for a description of elements of those constructs.

As used herein, "complementary" refers to polynucleotides that are capable of hybridizing, e.g. sense and anti-sense strands of DNA or self-complementary strands of RNA, due to complementarity of aligned nucleotides permitting C-G and A-T or A-U bonding.

As used herein "vector" means a DNA molecule capable of replication in a host cell and/or to which another DNA segment can be operatively linked so as to bring about replication of the attached segment. A plasmid is an exemplary vector.

As used herein a "transgenic" organism, e.g. plant or seed, is one whose genome has been altered by the incorporation of recombinant DNA comprising exogenous genetic material or additional copies of native genetic material, e.g. by transformation or recombination of the organism or an ancestral organism. Transgenic plants include progeny plants of an original plant derived from a transformation process including progeny of breeding transgenic plants with wild type plants or other transgenic plants. Crop plants of particular interest in the present invention include, but are not limited to maize, soybean, cotton, canola (rape), wheat, rice, sunflower, safflower and flax. Other crops of interest include plants producing vegetables, fruit, grass and wood.

### Recombinant DNA Constructs For Plant Transformation

Recombinant DNA constructs for producing looped, anti-sense RNA, gene suppression agents in transgenic plants can be readily prepared by those skilled in the art. Typically, such a DNA construct comprises as a minimum a promoter active in the tissue targeted for suppression, a transcribable DNA element having a sequence that is complementary to nucleotide sequence of a gene targeted for suppression and a transcription terminator element. The targeted gene element copied for use in transcribable DNA in the gene suppression construct can be a promoter element, an intron element, an exon element, a 5' UTR element, or a 3'UTR element. Although the minimum size of DNA copied from sequence of a gene targeted for suppression is believed to be about 21 or 23 nucleotides; larger nucleotide segments are preferred, e.g. up the full length of a targeted gene. Useful lengths of either DNA segment are in the range of 50 to 5000 nucleotides, say anti-sense-oriented DNA of 500 to 5000 nucleotides in length and complementary DNA elements can be 50 to 500 or more nucleotides in length. The DNA element can comprise multiple parts of a gene, e.g. nucleotides that are complementary to contiguous or separated gene elements of UTR, exon and intron. Such constructs may also comprise other regulatory elements, DNA encoding transit peptides, signal peptides, selective markers and screenable markers as desired.

With reference to Figure 1 there is schematically shown a recombinant DNA construct comprising a promoter element, an anti-sense-oriented DNA element (denoted "a/s DNA"), a complementary sense-oriented DNA element (denoted "s DNA") and DNA providing polyadenylation signals and site (denoted "polyA site"). The DNA construct is transcribed to RNA comprising an anti-sense-oriented RNA segment and a complementary RNA segment which is complementary to the 5'-most end of the anti-sense-oriented RNA segment. The 5' and 3' ends of the anti-sense RNA can self hybridize to form a double-stranded RNA segment that closes a loop of anti-sense-oriented RNA. For example, if the nucleotide sequence of the 5'-most end of the strand of transcribed anti-sense-oriented DNA is 5'-CGGCATA---, the sequence of the 3'-most end of the transcribed strand of the inverted repeat DNA will be ---TATGCCG-3' which is readily cloned from the source DNA providing the anti-sense element. With such sequences the loop of anti-sense-oriented RNA will extend from one side of a dsRNA segment, e.g.

5'-GCCGUAU--------
3'-CGGCAUA--------

The anti-sense-oriented DNA and its self-complementary DNA can be contiguous or separated by vector DNA, e.g. up to about 100 nucleotides or so of vector DNA separating restriction sites used for vector assembly.

Recombinant DNA constructs can be assembled using commercially available materials and methods known to those of ordinary skill in the art. A useful technology for building DNA constructs and vectors for transformation is the GATEWAY™ cloning technology (available from Invitrogen Life Technologies, Carlsbad, California) uses the site specific recombinase LR cloning reaction of the Integrase *att* system from bacterophage lambda vector construction, instead of restriction endonucleases and ligases. The LR cloning reaction is disclosed in U.S. Patents 5,888,732 and 6,277,608, U.S. Patent Application Publications 2001283529, 2001282319 and 20020007051, all of which are incorporated herein by reference. The GATEWAY™ Cloning Technology Instruction Manual which is also supplied by Invitrogen also provides concise directions for routine cloning of any desired DNA into a vector comprising operable plant expression elements.

An alternative vector fabrication method employs ligation-independent cloning as disclosed by Aslanidis, C. et al., Nucleic Acids Res., 18, 6069-6074, 1990 and Rashtchian, A. et al., Biochem., 206, 91-97,1992 where a DNA fragment with single-stranded 5' and 3' ends are ligated into a desired vector which can then be amplified *in vivo.*

Numerous promoters that are active in plant cells have been described in the literature. These include promoters present in plant genomes as well as promoters from other sources, including nopaline synthase (nos) promoter and octopine synthase (ocs) promoters carried on tumor-inducing plasmids of *Agrobacterium tumefaciens,* caulimovirus promoters such as the cauliflower mosaic virus or figwort mosaic virus promoters. For instance, see U.S. Patents 5,322,938 and 5,858,742 which disclose versions of the constitutive promoter derived from cauliflower mosaic virus (CaMV35S), US Patent 5,378,619 which discloses a Figwort Mosaic Virus (FMV) 35S promoter, U.S. Patent 5,420,034 which discloses a napin promoter, U.S. Patent 6,437,217 which discloses a maize RS81 promoter, U.S. Patent 5,641,876 which discloses a rice actin promoter, U.S. Patent 6,426,446 which discloses a maize RS324 promoter, U.S. Patent 6,429,362 which discloses a maize PR-1 promoter, U.S. Patent 6,232,526 which discloses a maize A3 promoter, U.S. Patent 6,177,611 which discloses constitutive maize promoters, U.S. Patent 6,433,252 which discloses a maize L3 oleosin promoter, U.S. Patent 6,429,357 which discloses a rice actin 2 promoter and intron, U.S. Patent 5,837,848 which discloses a root specific promoter, U.S. Patent 6,084,089 which discloses cold inducible promoters, U.S. Patent 6,294,714 which discloses light inducible promoters, U.S. Patent 6,140,078 which discloses salt inducible promoters, U.S. Patent 6,252,138 which discloses pathogen inducible promoters, U.S. Patent 6,175,060 which discloses phosphorus deficiency inducible promoters, U.S. Patent 6,635,806 which discloses a coixin promoter, U.S. 2002/0192813A1 which discloses 5', 3' and intron elements useful in the design of effective plant expression vectors, U.S.2004/0216189 A1 which discloses a maize chloroplast aldolase promoter, and U.S. 2004/0123347A1 which discloses water-deficit inducible promoters, all of which are incorporated herein by reference. These and numerous other promoters that function in plant cells are known to those skilled in the art and available for use in recombinant polynucleotides of the present invention to provide for expression of desired genes in transgenic plant cells.

Furthermore, the promoters may be altered to contain multiple "enhancer sequences" to assist in elevating gene expression. Such enhancers are known in the art. By including an enhancer sequence with such constructs, the expression of the selected protein may be enhanced. These enhancers often are found 5' to the start of transcription in a promoter that functions in eukaryotic cells, but can often be inserted upstream (5') or downstream (3') to the coding sequence. In some instances, these 5' enhancing elements are introns. Particularly useful as enhancers are the 5' introns of the rice actin 1 (see US Patent 5,641,876) and rice actin 2 genes, the maize alcohol dehydrogenase gene intron, the maize heat shock protein 70 gene intron (U.S. Patent 5,593,874) and the maize shrunken 1 gene.

In other aspects of the invention, sufficient expression in plant seed tissues is desired to effect improvements in seed composition. Exemplary promoters for use for seed composition modification include promoters from seed genes such as napin (U.S. 5,420,034), maize L3 oleosin (U.S. 6,433,252), zein Z27 (Russell et al. (1997) Transgenic Res. 6(2):157-166), globulin 1 (Belanger et al (1991) Genetics 129:863-872), glutelin 1 (Russell (1997) *supra*)*,* and peroxiredoxin antioxidant (Per1) (Stacy et al. (1996) Plant Mol Biol. 31(6):1205-1216).

Recombinant DNA constructs prepared in accordance with the invention will often include a 3' element that typically contains a polyadenylation signal and site, especially if the recombinant DNA is intended for protein expression as well as gene suppression. Well-known 3' elements include those from *Agrobacterium tumefaciens* genes such as *nos 3', tml 3', tmr 3', tms 3', ocs 3', tr7 3',* e.g. disclosed in U.S. 6,090,627, incorporated herein by reference; 3' elements from plant genes such as wheat (*Triticum aesevitum*) heat shock protein 17 (*Hsp17 3'*), a wheat ubiquitin gene, a wheat fructose-1,6-biphosphatase gene, a rice glutelin gene a rice lactate dehydrogenase gene and a rice beta-tubulin gene, all of which are disclosed in U.S. published patent application 2002/0192813 A1, incorporated herein by reference; and the pea (*Pisum sativum*) ribulose biphosphate carboxylase gene (*rbs 3'*), and 3' elements from the genes within the host plant.

The gene-suppressing recombinant DNA constructs can also be stacked with DNA imparting other traits of agronomic interest including DNA providing herbicide resistance or insect resistance such as using a gene from *Bacillus thuringensis* to provide resistance against lepidopteran, coliopteran, homopteran, hemiopteran, and other insects. Herbicides for which resistance is useful in a plant include glyphosate herbicides, phosphinothricin herbicides, oxynil herbicides, imidazolinone herbicides, dinitroaniline herbicides, pyridine herbicides, sulfonylurea herbicides, bialaphos herbicides, sulfonamide herbicides and glufosinate herbicides. Persons of ordinary skill in the art are enabled in providing stacked traits by reference to U.S. patent application publications 2003/0106096A1 and 2002/0112260A1 and U.S. Patents 5,034,322; 5,776,760; 6,107,549 and 6,376,754 and to insect/nematode/virus resistance by reference to U.S. Patents 5,250,515; 5,880,275; 6,506,599; 5,986,175 and U.S. Patent Application Publication 2003/0150017 A1, all of which are incorporated herein by reference.

**Transformation Methods -** Numerous methods for transforming plant cells with recombinant DNA are known in the art and may be used in the present invention. Two commonly used methods for plant transformation are *Agrobacterium-mediated* transformation and microprojectile bombardment. Microprojectile bombardment methods are illustrated in U.S. Patents 5,015,580 (soybean); 5,550,318 (corn); 5,538,880 (corn); 5,914,451 (soybean); 6,160,208 (corn); 6,399,861 (corn) and 6,153,812 (wheat) and *Agrobacterium*-mediated transformation is described in U.S. Patents 5,159,135 (cotton); 5,824,877 (soybean); 5,591,616 (corn); and 6,384,301 (soybean), all of which are incorporated herein by reference. For *Agrobacterium tumefaciens* based plant transformation system, additional elements present on transformation constructs will include T-DNA left and right border sequences to facilitate incorporation of the recombinant polynucleotide into the plant genome.

In general it is useful to introduce recombinant DNA randomly, i.e. at a nonspecific location, in the genome of a target plant line. In special cases it may be useful to target recombinant DNA insertion in order to achieve site-specific integration, e.g. to replace an existing gene in the genome, to use an existing promoter in the plant genome, or to insert a recombinant polynucleotide at a predetermined site known to be active for gene expression. Several site specific recombination systems exist which are known to function implants include cre-lox as disclosed in U.S. Patent 4,959,317 and FLP-FRT as disclosed in U.S. Patent 5,527,695, both incorporated herein by reference.

Transformation methods of this invention are preferably practiced in tissue culture on media and in a controlled environment. "Media" refers to the numerous nutrient mixtures that are used to grow cells *in vitro,* that is, outside of the intact living organism. Recipient cell targets include, but are not limited to, meristem cells, callus, immature embryos and gametic cells such as microspores, pollen, sperm and egg cells. It is contemplated that any cell from which a fertile plant may be regenerated is useful as a recipient cell. Callus may be initiated from tissue sources including, but not limited to, immature embryos, seedling apical meristems, microspores and the like. Cells capable of proliferating as callus are also recipient cells for genetic transformation. Practical transformation methods and materials for making transgenic plants of this invention, e.g. various media and recipient target cells, transformation of immature embryos and subsequent regeneration of fertile transgenic plants are disclosed in U.S. Patents 6,194,636 and 6,232,526, which are incorporated herein by reference.

The seeds of transgenic plants can be harvested from fertile transgenic plants and be used to grow progeny generations of transformed plants of this invention including hybrid plants line for screening of plants having an enhanced agronomic trait. In addition to direct transformation of a plant with a recombinant DNA, transgenic plants can be prepared by crossing a first plant having a recombinant DNA with a second plant lacking the DNA. For example, recombinant DNA can be introduced into first plant line that is amenable to transformation to produce a transgenic plant which can be crossed with a second plant line to introgress the recombinant DNA into the second plant line. A transgenic plant with recombinant DNA providing an enhanced agronomic trait, e.g. enhanced yield, can be crossed with transgenic plant line having other recombinant DNA that confers another trait, e.g. herbicide resistance or pest resistance, to produce progeny plants having recombinant DNA that confers both traits. Typically, in such breeding for combining traits the transgenic plant donating the additional trait is a male line and the transgenic plant carrying the base traits is the female line. The progeny of this cross will segregate such that some of the plants will carry the DNA for both parental traits and some will carry DNA for one parental trait; such plants can be identified by markers associated with parental recombinant DNA Progeny plants carrying DNA for both parental traits can be crossed back into the female parent line multiple times, e.g. usually 6 to 8 generations, to produce a progeny plant with substantially the same genotype as one original transgenic parental line but for the recombinant DNA of the other transgenic parental line

In the practice of transformation DNA is typically introduced into only a small percentage of target cells in any one transformation experiment. Marker genes are used to provide an efficient system for identification of those cells that are stably transformed by receiving and integrating a transgenic DNA construct into their genomes. Preferred marker genes provide selective markers which confer resistance to a selective agent, such as an antibiotic or herbicide. Any of the herbicides to which plants of this invention may be resistant are useful agents for selective markers. Potentially transformed cells are exposed to the selective agent. In the population of surviving cells will be those cells where, generally, the resistance-conferring gene is integrated and expressed at sufficient levels to permit cell survival. Cells may be tested further to confirm stable integration of the exogenous DNA. Commonly used selective marker genes include those conferring resistance to antibiotics such as kanamycin and paromomycin (*nptII*)*,* hygromycin B (*aph IV*) and gentamycin (*aac3* and *aac*C4) or resistance to herbicides such as glufosinate (*bar* or *pat*) and glyphosate (*aroA* or EPSPS). Examples of such selectable are illustrated in U.S. Patents 5,550,318; 5,633,435; 5,780,708 and 6,118,047, all of which are incorporated herein by reference. Screenable markers which provide an ability to visually identify transformants can also be employed, e.g., a gene expressing a colored or fluorescent protein such as a luciferase or green fluorescent protein (GFP) or a gene expressing a *beta*-glucuronidase or *uid*A gene (GUS) for which various chromogenic substrates are known.

Cells that survive exposure to the selective agent, or cells that have been scored positive in a screening assay, may be cultured in regeneration media and allowed to mature into plants. Developing plantlets can be transferred to plant growth mix, and hardened off, *e.g*., in an environmentally controlled chamber at about 85% relative humidity, 600 ppm CO₂, and 25-250 microeinsteins m⁻² s⁻¹ of light, prior to transfer to a greenhouse or growth chamber for maturation. Plants are regenerated from about 6 weeks to 10 months after a transformant is identified, depending on the initial tissue. Plants may be pollinated using conventional plant breeding methods known to those of skill in the art and seed produced, e.g. self-pollination is commonly used with transgenic corn. The regenerated transformed plant or its progeny seed or plants can be tested for expression of the recombinant DNA and screened for the presence of enhanced agronomic trait.

### Transgenic Plants and Seeds

Transgenic plant seed provided by this invention are grown to generate transgenic plants having an enhanced trait as compared to a control plant. Such seed for plants with enhanced agronomic trait is identified by screening transformed plants or progeny seed for enhanced trait. For efficiency a screening program is designed to evaluate multiple transgenic plants (events) comprising the recombinant DNA , e.g. multiple plants from 2 to 20 or more transgenic events.
Transgenic plants grown from transgenic seed provided herein demonstrate improved agronomic traits that contribute to increased yield or other trait that provides increased plant value, including, for example, improved seed quality. Of particular interest are plants having enhanced yield resulting from improved plant growth and development, stress tolerance, improved seed development, higher light response, improved flower development, or improved carbon and/or nitrogen metabolism

Many transgenic events which survive to fertile transgenic plants that produce seeds and progeny plants will not exhibit an enhanced agronomic trait. Screening is necessary to identify the transgenic plant having enhanced agronomic traits from populations of plants transformed as described herein by evaluating the trait in a variety of assays to detect an enhanced agronomic trait. These assays also may take many forms, including but not limited to, analyses to detect changes in the chemical composition, biomass, physiological properties, morphology of the plant.

The following examples illustrate aspects of the invention.

### Example 1

This example illustrates preparation of a transformation vector useful for inserting a recombinant DNA construct of this invention into a transgenic plant to practice a method of this invention.

The *LKR*/*SDH* gene encodes a pre-protein for lysine ketoglutarate reductase (LKR) and saccharopine dehydrogenase (SDH) which are enzymes in a lysine catabolic pathway. Suppression of *LKR* is manifest in modification, e.g. increase, of lysine content. Suppression of *LKR* is effected by expressing in a plant a recombinant DNA construct that produces a stabilized anti-sense RNA transcribed from anti-sense-oriented *LKR* DNA and sense-oriented *LKR* DNA which forms a loop of anti-sense-oriented RNA.

A transformation vector is prepared comprising two transcription units between right and left borders from *Agrobacterium tumefaciens.* One transcription unit for a marker comprised:
(a) DNA of a rice actin promoter and rice actin intron,
(b) DNA of a chloroplast transit peptide from *Arabidopsis* EPSPS
(c) DNA *of A. tumefaciens aroA* (a glyphosate-resistant marker), and
(d) DNA of *A. tumefaciens NOS* terminator,

The other transcription unit for *LKR* gene suppression comprised:
(a) DNA of *Zea mays GLB1* promoter,
(b) DNA of a *Zea mays ADH1* intron,
(c) Anti-sense-oriented DNA fragment of *Zea mays LKR,*
(d) Sense-oriented DNA fragment of *Zea mays LKR,* and
(e) DNA of *Zea mays GLB1* terminator.

SEQ ID NO: 1 is DNA sequence of a transformation vector comprising the above-described marker and gene suppression elements. See Table 1 below for a description of the elements of the transformation vector contained within SEQ ID NO:1 .

**Table 1.**

| **Bases of SEQ ID NO:1** | **Description of DNA segment** |
|---|---|
| 1-357 | *A. tumefaciens* right border |
| 376-1774 | DNA of a rice actin promoter and rice actin intron |
| 1784-2011 | DNA of *A. tumefaciens* EPSPS chloroplast transit peptide |
| 2012-3379 | DNA of *A. tumefaciens aroA* (glyphosate-resistant marker) |
| 3395-3647 | DNA of *A. tumefaciens NOS* terminator |
| 3691-4686 | DNA of *Zea mays Glb1* terminator |
| 4692-5145 | Sense-oriented DNA element from *Zea mays LKR* |
| 5152-6118 | Anti-sense-oriented DNA element from *Zea mays LKR* |
| 6123-6680 | DNA of a *Zea mays ADH1* intron |
| 6687-8082 | DNA of *Zea mays GLB1* promoter |
| 8149-8590 | *A. tumefaciens* left border |

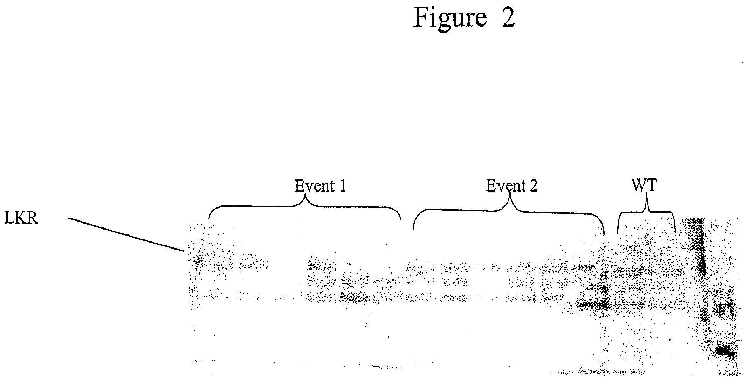

A vector prepared with the elements listed in Table 1 was used to transform corn plant tissue. Transgenic corn plants were obtained by *Agrobacterium-*mediated transformation. Transgenic plants from two separate transgenic insertion events were grown to produce F1 seed. Six mature seeds from each event were analyzed to determine success of transformation and suppression of *LKR.* The mature transgenic seeds were dissected to extract protein which was analyzed by Western analysis. With reference to Figure 2, seed from one of the events showed no reduction in *LKR* as compared to wild type; and seed from the other event was shown to be segregating (1:1 hemizygous:wild type) as three of the six seeds showed substantial reduction in *LKR* as compared to wild type.

### Example 2

This example illustrates a wide scope of embodiments of transformation vectors useful for inserting a recombinant DNA construct of this invention into a transgenic plant to practice a method of this invention. Transformation vectors were prepared using the following DNA elements where:
(a) "pGcx" refers to DNA for a promoter derived from a gamma coixin gene from *Coix lacryma-jobi*;
(b) "pZ27" refers to DNA for a promoter derived from a gamma zein gene from *Zea mays*;
(c) "pZ27t" refers to DNA for a truncated promoter having 59 nucleotides leader sequence deleted from the 3' region of pZ27;
(d) "Z19as" refers to DNA for an antisense-oriented segment of 351 nucleotides from the coding sequence of a 19 kilo dalton alpha zein gene from *Zea mays*;
(e) "Z19s" refers to DNA for a sense-oriented segment of 351 nucleotides from the coding sequence of a 19 kilo dalton alpha zein gene from *Zea mays,* which is an inverted repeat of Z19as;
(f) "Z22as" refers to DNA for an antisense-oriented segment of 789 nucleotides from the coding sequence of a 22 kilo dalton alpha zein gene from *Zea mays*;
(g) "Z22asL" refers to DNA for an antisense-oriented segment of 785 nucleotides from the coding sequence of a 22 kilo dalton alpha zein gene from *Zea mays*;
(h) "Z22asSI" refers to DNA for an antisense-oriented segment of 789 nucleotides from the coding sequence of a 22 kilo dalton alpha zein gene from *Zea mays* having a 520 nucleotide long spliceable intron from a GB1 gene intron 3 from *Zea mays* inserted in the unpaired region;
(i) "Z22s" refers to DNA for a sense-oriented segment of 289 nucleotides from the coding sequence of a 22 kilo dalton alpha zein gene from *Zea mays,* which is an inverted repeat of the 5' end of Z22as; and
(j) "TE9" refers to DNA for a sense oriented polyadenylation signal and site element from an RbcS2 gene from *Pisum sativum.*

With reference to Table 2 and SEQ ID NO:2 a transformation vector comprising "construct 2a" was made in the manner of Example 1 except that the transcription unit for LKR gene suppression was replaced by a transcription unit comprising the elements illustrated in the following schematic:
"Construct 2a" pZ27 - Z19as - Z22asL - Z22s - Z19s - TE9

**Table 2**

| **Bases of SEQ ID NO:2** | **description of DNA segment** |
|---|---|
| 1-357 | *A. tumefaciens* right border |
| 376-1774 | DNA of a rice actin promoter and rice actin intron |
| 1784-2011 | DNA of *A. tumefaciens* EPSPS chloroplast transit peptide |
| 2012-3379 | DNA of *A. tumefaciens aroA* (glyphosate-resistant marker) |
| 3395-3647 | DNA of *A. tumefaciens NOS* terminator |
| 3479-4391 | DNA of *Pisum sativum RbcS2* terminator |
| 4398-4748 | DNA for Z19s |
| 4755-5043 | DNA for Z22s |
| 5050-5835 | DNA of Z22asL |
| 5842-6192 | DNA of Z19as |
| 6204-7305 | DNA of *Zea mays Z27* promoter |
| 7353-7794 | *A. tumefaciens* left border |

Corn callus was transformed and events with a single copy of the transformation vector were selected for growth into plants. Seed from plants grown from 26 of 29 single copy events showed substantial reduction of the 19 kilo dalton alpha zeins and the 22 kilo Dalton alpha zeins.

Other transformation vectors were made in a similar manner using the elements illustrated in the following Table 3.

**Table 3**

| | |
|---|---|
| Construct 2b1 | pGcx - Z19as - Z22asSI - Z22s - Z19s - TE9 |
| Construct 2b2* | pGcx - Z19as - Z22asSI - Z22s - Z19s - TE9 |
| Construct 2c | pZ27 - Z19as - Z22asSI - Z22s - Z19s - TE9 |
| Construct 2d | PZ27t - Z19as - Z22asSI - Z22s - Z19s - TE9 |
| Construct 2e | PZ27 - Z19as - Z22asL - Z19s - TE9 |

| | |
|---|---|
| * construct 2b2 was inserted into a transformation vector that also included a transcription unit for expressing another gene having a promoter contiguous to pGcx. | |

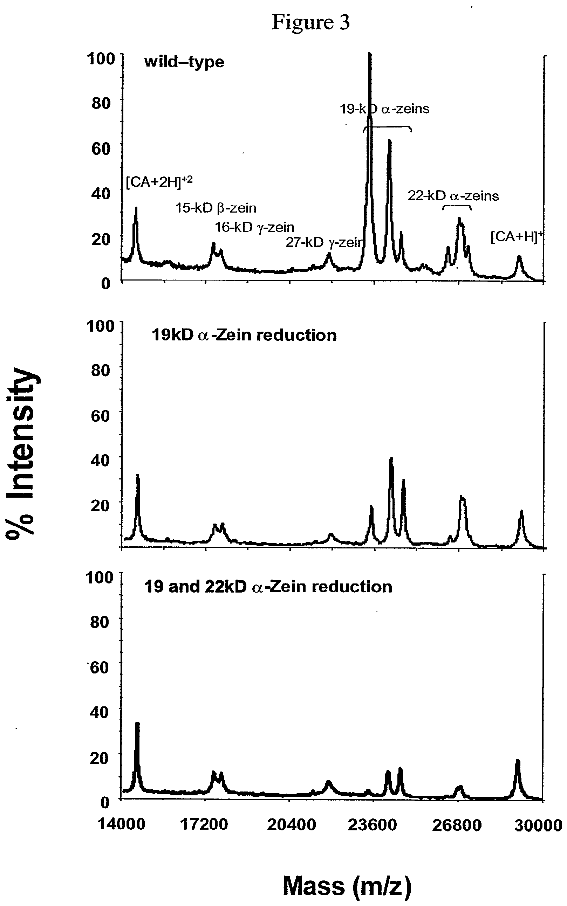

The efficiency of suppressing the alpha zeins in seeds produced by plants grown from single copy events is reported in Table 4 which reports the number of transgenic events with reduction of zeins as compared to the total number of transgenic events generated in each construct tested. The zein reduction phenotype is observed by MALDI-TOF MS (Matrix-Assisted-Laser-Desorption Ionization Time-Of-Flight Mass Spectrometry) analysis. Figure 3 is illustrates typical spectra evidencing zein reduction.

**Table 4**

| Construct | 19 kD zein | 19 and 22 kD zein |
|---|---|---|
| 2a | 26/29 | 26/29 |
| 2b1 | 0/21 | 0/21 |
| 2b2 | 5/7 | 0/7 |
| 2c | 20/21 | 18/21 |
| 2d | 7/8 | 1/8 |
| 2e | 12/14 | 2/14 |

All of the materials and methods disclosed and claimed herein can be made and used without undue experimentation as instructed by the above disclosure. Although the materials and methods ofthis invention have been described in terms of preferred embodiments and illustrative examples, it will be apparent to those of skill in the art that variations may be applied to the materials and methods described herein without departing from the concept, spirit and scope of the invention. All such similar substitutes and modifications apparent to those skilled in the art are deemed to be within the spirit, scope and concept of the invention as defined by the appended claims.

A recombinant DNA construct for suppression of at least one target gene which comprises in 5' to 3' order a promoter element operably linked to an anti-sense-oriented DNA element from at least one target gene and a sense-oriented DNA element, wherein the sense-oriented DNA element is shorter than the anti-sense-oriented DNA element, and sense-oriented RNA transcribed by the sense-oriented DNA is complementary to the 5'-most end of anti-sense-oriented RNA transcribed by the anti-sense-oriented DNA element, wherein said transcribed RNA forms a into a loop of anti-sense-oriented RNA for suppressing said at least one target gene.
A recombinant DNA construct as defined above, wherein said anti-sense-oriented DNA element comprises, in series, segments from two or more genes targeted for suppression.
A recombinant DNA construct as defined above, wherein said sense-oriented DNA element is from one gene targeted for suppression and said anti-sense-oriented DNA is from two or more genes targeted for suppression.
A recombinant DNA construct as defined above, wherein said sense-oriented DNA element and said anti-sense-oriented DNA are from a single gene targeted for suppression.
A recombinant DNA construct as defined above, wherein said loop of anti-sense-oriented RNA is closed with a segment of double-stranded RNA.

A recombinant DNA construct as defined above, wherein one strand of said segment of double-stranded RNA is identical to mRNA from a gene targeted for suppression. A recombinant DNA construct as defined above, wherein said segment of double-stranded RNA is transcribed from DNA which is not from a gene targeted for suppression.
A method for generating anti-sense-oriented RNA in an organism for suppression of a target gene, said method comprising providing in cells of said organism a recombinant DNA construct which is transcribed to RNA that forms a loop of anti-sense-oriented RNA.
A method as defined above, wherein said anti-sense-oriented DNA element comprises, in series, segments from two or more genes targeted for suppression. A method as defined above, wherein said sense-oriented DNA element is from one gene targeted for suppression and said anti-sense-oriented DNA is from two or more genes targeted for suppression.
A method as defined above, wherein said sense-oriented DNA element and said anti-sense-oriented DNA are from a single gene targeted for suppression. A method as defined above, wherein said loop of anti-sense-oriented RNA is closed with a segment of double-stranded RNA.
A method as defined above, wherein one strand of said segment of double-stranded RNA is identical to mRNA from a gene targeted for suppression.
A method as defined above, wherein said segment of double-stranded RNA is transcribed from DNA which is not from a gene targeted for suppression.

## Claims

1. A recombinant RNA molecule for suppression of at least one target gene which comprises in 5' to 3' order an anti-sense-oriented RNA segment from at least one target gene and a sense-oriented RNA segment, wherein the sense-oriented RNA segment is shorter than the anti-sense-oriented RNA segment, and the sense-oriented RNA segment is complementary to the 5 '-most end of the anti-sense-oriented RNA segment, and the RNA molecule comprises a loop of anti-sense-oriented RNA for suppressing said at least one target gene.

2. The recombinant RNA molecule of claim 1 wherein said anti-sense-oriented RNA segment comprises, in series, segments from two or more genes targeted for suppression.

3. The recombinant RNA molecule of claim 1 wherein said sense-oriented RNA segment is from one gene targeted for suppression and said anti-sense-oriented RNA segment is from two or more genes targeted for suppression.

4. The recombinant RNA molecule of claim 1 wherein said sense-oriented RNA segment and said anti-sense-oriented RNA segment are from a single gene targeted for suppression.

5. The recombinant RNA molecule of claim 1 wherein said loop of anti-sense-oriented RNA is closed with a segment of double-stranded RNA.

6. The recombinant RNA molecule of claim 5 wherein one strand of said segment of double-stranded RNA is identical to mRNA from a gene targeted for suppression.

7. The recombinant RNA molecule of claim 5 wherein said segment of double-stranded RNA is transcribed from DNA which is not from a gene targeted for suppression.

8. The recombinant RNA molecule of claim 1, wherein the sense-oriented RNA segment is not more than about one-half the length of the anti-sense-oriented RNA segment.

9. The recombinant RNA molecule of claim 1, wherein the sense-oriented RNA segment is not more than about one-third the length of the anti-sense-oriented RNA segment.

10. The recombinant RNA molecule of claim 1, wherein the sense-oriented RNA segment is not more than about one-quarter the length of the anti-sense-oriented RNA segment.

11. The recombinant RNA molecule of claim 1, wherein the sense-oriented RNA segment comprises from 50 to 500 nucleotides.

12. A method for generating anti-sense-oriented RNA in a plant for suppression of a target gene, said method comprising providing in cells of said-plant a recombinant RNA molecule according to any one of claims 1 to 11.


SEQUENCE LISTING

<110>  Monsanto Technology, LLC

<120>  Recombinant DNA for Gene Suppression

<130>  38-15 (53428)B

<150>  EP 11160073.0
<151>  2005-02-10

<150>  EP 05713459.5
<151>  2005-02-10

<150>  US 60/543,157
<151>  2004-02-10

<150>  US 60/543,187
<151>  2004-02-10

<150>  US 60/600,859
<151>  2004-08-11

<160>  2

<170>  PatentIn version 3.2

<210>  1
<211>  8590
<212>  DNA
<213>  Artificial

<220>
<223>  recombinant DNA construct in plasmid between Agrabacterium
       borders

<400>  1
aggatttttc ggcgctgcgc tacgtccgcg accgcgttga gggatcaagc cacagcagcc      60

cactcgacct tctagccgac ccagacgagc caagggatct ttttggaatg ctgctccgtc     120

gtcaggcttt ccgacgtttg ggtggttgaa cagaagtcat tatcgcacgg aatgccaagc     180

actcccgagg ggaaccctgt ggttggcatg cacatacaaa tggacgaacg gataaacctt     240

ttcacgccct tttaaatatc cgattattct aataaacgct cttttctctt aggtttaccc     300

gccaatatat cctgtcaaac actgatagtt taaactgaag gcgggaaacg acaatctgat     360

ccccatcaag cttactcgag gtcattcata tgcttgagaa gagagtcggg atagtccaaa     420

ataaaacaaa ggtaagatta cctggtcaaa agtgaaaaca tcagttaaaa ggtggtataa     480

agtaaaatat cggtaataaa aggtggccca aagtgaaatt tactcttttc tactattata     540

aaaattgagg atgtttttgt cggtactttg atacgtcatt tttgtatgaa ttggttttta     600

agtttattcg cttttggaaa tgcatatctg tatttgagtc gggttttaag ttcgtttgct     660

tttgtaaata cagagggatt tgtataagaa atatctttag aaaaacccat atgctaattt     720

gacataattt ttgagaaaaa tatatattca ggcgaattct cacaatgaac aataataaga     780

ttaaaatagc tttcccccgt tgcagcgcat gggtattttt tctagtaaaa ataaaagata 840

aacttagact caaaacattt acaaaaacaa cccctaaagt tcctaaagcc caaagtgcta 900

tccacgatcc atagcaagcc cagcccaacc caacccaacc cagcccaccc cagtccagcc 960

aactggacaa tagtctccac acccccccac tatcaccgtg agttgtccgc acgcaccgca 1020

cgtctcgcag ccaaaaaaaa aaagaaagaa aaaaaagaaa aagaaaaaac agcaggtggg 1080

tccgggtcgt gggggccgga aacgcgagga ggatcgcgag ccagcgacga ggccggccct 1140

ccctccgctt ccaaagaaac gccccccatc gccactatat acataccccc ccctctcctc 1200

ccatcccccc aaccctacca ccaccaccac caccacctcc acctcctccc ccctcgctgc 1260

cggacgacga gctcctcccc cctccccctc cgccgccgcc gcgccggtaa ccaccccgcc 1320

cctctcctct ttctttctcc gttttttttt ccgtctcggt ctcgatcttt ggccttggta 1380

gtttgggtgg gcgagaggcg gcttcgtgcg cgcccagatc ggtgcgcggg aggggcggga 1440

tctcgcggct ggggctctcg ccggcgtgga tccggcccgg atctcgcggg gaatggggct 1500

ctcggatgta gatctgcgat ccgccgttgt tgggggagat gatggggggt ttaaaatttc 1560

cgccgtgcta aacaagatca ggaagagggg aaaagggcac tatggtttat atttttatat 1620

atttctgctg cttcgtcagg cttagatgtg ctagatcttt ctttcttctt tttgtgggta 1680

gaatttgaat ccctcagcat tgttcatcgg tagtttttct tttcatgatt tgtgacaaat 1740

gcagcctcgt gcggagcttt tttgtaggta gaagtgatca accatggcgc aagttagcag 1800

aatctgcaat ggtgtgcaga acccatctct tatctccaat ctctcgaaat ccagtcaacg 1860

caaatctccc ttatcggttt ctctgaagac gcagcagcat ccacgagctt atccgatttc 1920

gtcgtcgtgg ggattgaaga agagtgggat gacgttaatt ggctctgagc ttcgtcctct 1980

taaggtcatg tcttctgttt ccacggcgtg catgcttcac ggtgcaagca gccggcccgc 2040

aaccgcccgc aaatcctctg gcctttccgg aaccgtccgc attcccggcg acaagtcgat 2100

ctcccaccgg tccttcatgt tcggcggtct cgcgagcggt gaaacgcgca tcaccggcct 2160

tctggaaggc gaggacgtca tcaatacggg caaggccatg caggcgatgg gcgcccgcat 2220

ccgtaaggaa ggcgacacct ggatcatcga tggcgtcggc aatggcggcc tcctggcgcc 2280

tgaggcgccg ctcgatttcg gcaatgccgc cacgggctgc cgcctgacga tgggcctcgt 2340

cggggtctac gatttcgaca gcaccttcat cggcgacgcc tcgctcacaa agcgcccgat 2400

gggccgcgtg ttgaacccgc tgcgcgaaat gggcgtgcag gtgaaatcgg aagacggtga 2460

ccgtcttccc gttaccttgc gcgggccgaa gacgccgacg ccgatcacct accgcgtgcc 2520

gatggcctcc gcacaggtga agtccgccgt gctgctcgcc ggcctcaaca cgcccggcat 2580

cacgacggtc atcgagccga tcatgacgcg cgatcatacg gaaaagatgc tgcagggctt 2640

tggcgccaac cttaccgtcg agacggatgc ggacggcgtg cgcaccatcc gcctggaagg 2700

ccgcggcaag ctcaccggcc aagtcatcga cgtgccgggc gacccgtcct cgacggcctt 2760

cccgctggtt gcggccctgc ttgttccggg ctccgacgtc accatcctca acgtgctgat 2820

gaaccccacc cgcaccggcc tcatcctgac gctgcaggaa atgggcgccg acatcgaagt 2880

catcaacccg cgccttgccg gcggcgaaga cgtggcggac ctgcgcgttc gctcctccac 2940

gctgaagggc gtcacggtgc cggaagaccg cgcgccttcg atgatcgacg aatatccgat 3000

tctcgctgtc gccgccgcct tcgcggaagg ggcgaccgtg atgaacggtc tggaagaact 3060

ccgcgtcaag gaaagcgacc gcctctcggc cgtcgccaat ggcctcaagc tcaatggcgt 3120

ggattgcgat gagggcgaga cgtcgctcgt cgtgcgtggc cgccctgacg gcaaggggct 3180

cggcaacgcc tcgggcgccg ccgtcgccac ccatctcgat caccgcatcg ccatgagctt 3240

cctcgtcatg ggcctcgtgt cggaaaaccc tgtcacggtg gacgatgcca cgatgatcgc 3300

cacgagcttc ccggagttca tggacctgat ggccgggctg ggcgcgaaga tcgaactctc 3360

cgatacgaag gctgcctgat gagctcgaat tcccgatcgt tcaaacattt ggcaataaag 3420

tttcttaaga ttgaatcctg ttgccggtct tgcgatgatt atcatataat ttctgttgaa 3480

ttacgttaag catgtaataa ttaacatgta atgcatgacg ttatttatga gatgggtttt 3540

tatgattaga gtcccgcaat tatacattta atacgcgata gaaaacaaaa tatagcgcgc 3600

aaactaggat aaattatcgc gcgcggtgtc atctatgtta ctagatcggg gatgggggat 3660

ccactagtga tatccgtcga gtggcggccg cgttttatga ataataataa tgcatatctg 3720

tgcattacta cctgggatac aagggcttct ccgccataac aaattgagtt gcgatgctga 3780

gaacgaacgg ggaagaaagt aagcgccgcc caaaaaaaac gaacatgtac gtcggctata 3840

gcaggtgaaa gttcgtgcgc caatgaaaag ggaacgatat gcgttgggta gttgggatac 3900

ttaaatttgg agagtttgtt gcatacacta atccactaaa gttgtctatc tttttaacag 3960

ctctaggcag gatataagat ttatatctaa tctgttggag ttgcttttag agtaactttt 4020

ctctctgttt cgtttatagc cgattagcac aaaattaaac taggtgacga gaaataaaga 4080

aaaacggagg cagtaaaaaa tacccaaaaa aatacttgga gatttttgtc tcaaaattat 4140

cttctaattt taaaagctac atattaaaaa tactatatat taaaaatact tcgagatcat 4200

tgcttgggat gggcagggcc aatagctaat tgctaaggat gggctatatt tatgtatcgt 4260

ctgaaacatg taggggctaa tagttagatg actaatttgc tgtgttcgta cggggtgctg 4320

tttgagccta gcgatgaagg gtcatagttt catacaagaa ctcacttttg gttcgtctgc 4380

tgtgtctgtt ctcagcgtaa cggcatcaat ggatgccaaa ctccgcaagg ggacaaatga 4440

agaagcgaag agattataga acacgcacgt gtcattattt atttatggac ttgcctcagt 4500

agcttacagc atcgtacccg cacgtacata ctacagagcc acacttattg cactgcctgc 4560

cgcttacgta catagttaac acgcagagag gtatatacat acacgtccaa cgtctccact 4620

caggctcatg ctacgtacgc acgtcggtcg cgcgccaccc tctcgttgct tcctgctcgt 4680

tttggcgaat tccgatttgg caagtgttcc agagcaaaag ctggaagctc tcgtagtctg 4740

agcctctttg ctgattcata caagttatga ccatctacat ggatcgtctc accaagaaat 4800

ttgtagactg caggattttt ccctgaccgg agtgcaccag ctgggttcca actgaattta 4860

taggcaagcg gattgtttgc tgcagctgga gatggcaatc caccacagta agatgtaaat 4920

gcctttattt ttccctttcg tgcatgagct tcatcaatca tcttcattga catcaagtga 4980

tctatgccag gatctaggcc catttcacaa agtatagtta cacctgcatc tttggcagct 5040

tggctcaagt ttgacatgga ttcatcaaca tagcttgccg ttaccatgtg cttcttcaac 5100

tctatgcata ctcctgcaat ggcagcatga aaactagcag gcagcaccgg ttggacatca 5160

ttgagacagc tggaggttca tttcacttgg ttagatgtga agttggacaa agcacggatg 5220

atatgtcgta ctcagagctt gaagtaggag cagatgatac tgccacattg gataaaatta 5280

ttgattcctt gacttcttta gctaatgaac atggtggaga tcacgatgcc gggcaagaaa 5340

ttgaattagc tctgaagata ggaaaagtca atgagtatga aactgacgtc acaattgata 5400

aaggagggcc aaagatttta attcttggag ctggaagagt ctgtcggcca gctgctgagt 5460

ttctggcatc ttacccagac atatgtacct atggtgttga tgaccatgat gcagatcaaa 5520

ttcatgttat cgtggcatct ttgtatcaaa aagatgcaga agagacagtt gatggtattg 5580

aaaatacaac tgctacccag cttgatgttg ctgatattgg aagcctttca gatcttgttt 5640

ctcaggttga ggttgtaatt agcttgctgc ctgctagttt tcatgctgcc attgcaggag 5700

tatgcataga gttgaagaag cacatggtaa cggcaagcta tgttgatgaa tccatgtcaa 5760

acttgagcca agctgccaaa gatgcaggtg taactatact ttgtgaaatg ggcctagatc 5820

ctggcataga tcacttgatg tcaatgaaga tgattgatga agctcatgca cgaaagggaa 5880

aaataaaggc atttacatct tactgtggtg gattgccatc tccagctgca gcaaacaatc 5940

cgcttgccta taaattcagt tggaacccag ctggtgcact ccggtcaggg aaaaatcctg 6000

cagtctacaa atttcttggt gagacgatcc atgtagatgg tcataacttg tatgaatcag 6060

caaagaggct cagactacga gagcttccag cttttgctct ggaacacttg ccaaatcggg 6120

atccgcagct gcacgggtcc aggaaagcaa tcgcatagtc aagctaaatc atcaagatgc 6180

aaacttttcg cccttgctaa acacggtaaa attcgaatgg acatgtgtgg agcagcaaag 6240

gccttacgtc cgagaaacag ggccactcaa cgagttagtt aaattcaaag aaagaaacgc 6300

ctccttgcaa gttgcaacat tcttagatca tactgatgaa aatgacgtct ttcattaaag 6360

aacaggaaag atagatcttt gctcaatatc gtatgatgtg ttcagccaga ctgtcggatg 6420

gaccacacgg taatagcagt gctggacgat gttacatcga gaaagattac tagccttttc 6480

atgggagtga aggatataaa agaaataagt tcaccacgat tgcaggatag catacaagat 6540

cagcgccact gcggcactgt tcatcgaaaa aaaaactgtg gacgaagcta gctttcccca 6600

aaattactca acgaatcata aaccaagatt agtcagatca agagacagag gagaaacaag 6660

gcggaccttt gcacttgatc ggatccttgg gttggctgta tgcagaacta aagcggaggt 6720

ggcgcgcatt tataccagcg ccgggccctg gtacgtggcg cggccgcgcg gctacgtgga 6780

ggaaggctgc gtggcagcag acacacgggt cgccacgtcc cgccgtactc tccttaccgt 6840

gcttatccgg gctccggctc ggtgcacgcc agggtgtggc cgcctctgag cagactttgt 6900

cgtgttccac agtggtgtcg tgttccgggg actccgatcc gcggcgagcg accgagcgtg 6960

taaaagagtt cctactaggt acgttcattg tatctggacg acgggcagcg gacaatttgc 7020

tgtaagagag gggcagtttt tttttagaaa aacagagaat tccgttgagc taattgtaat 7080

tcaacaaata agctattagt tggttttagc ttagattaaa gaagctaacg actaatagct 7140

aataattagt tggtctatta gttgactcat tttaaggccc tgtttcaatc tcgcgagata 7200

aactttagca gctatttttt agctactttt agccatttgt aatctaaaca ggagagctaa 7260

tggtggtaat tgaaactaaa ctttagcact tcaattcata tagctaaagt ttagcaggaa 7320

gctaaacttt atcccgtgag attgaaacgg ggcctaaatc tctcagctat ttttgatgca 7380

aattactgtc actactggaa tcgagcgctt tgccgagtgt caaagcctga aaaacactcc 7440

gtaaagactt tgcctagtgt gacactcgac aaagagatct cgacgaacag tacatcgaca 7500

acggcttctt tgtcgagtac tttttatcgg acacttgaca aagtctttgt cgagtgaact 7560

acattgaaac tctatgattt tatgtgtagg tcacttaggt ttctacacat agtacgtcac 7620

aactttaccg aaacattatc aaatttttat cacaacctct atatatgata tcatgacatg 7680

tggacaagtt tcattaattt ctgactttat ttgtgtttta tacaattttt aaacaactag 7740

ataacaagtt cacggtcatg tttagtgagc atggtgcttg aagattctgg tctgcttctg 7800

aaatcggtcg taacttgtgc tagataacat gcatatcatt tattttgcat gcacggtttt 7860

ccatgtttcg agtgacttgc agtttaaatg tgaattttcc gaagaaattc aaataaacga 7920

actaaatcta atatttatag aaaacatttt tgtaaatatg taattgtgcc aaaatggtac 7980

atgtagatct acatagtgta ggaacatacc acaaaaagtt tggttggcaa aataaaaaaa 8040

ataaaatata ctttatccga gtgtccaagg tatggcactc ggcccgggtg gccaagctta 8100

ctagcccggg cgcgccttaa ttaagcggcc gcatcgatcg tgaagtttct catctaagcc 8160

cccatttgga cgtgaatgta gacacgtcga aataaagatt tccgaattag aataatttgt 8220

ttattgcttt cgcctataaa tacgacggat cgtaatttgt cgttttatca aaatgtactt 8280

tcattttata ataacgctgc ggacatctac atttttgaat tgaaaaaaaa ttggtaatta 8340

ctctttcttt ttctccatat tgaccatcat actcattgct gatccatgta gatttcccgg 8400

acatgaagcc atttacaatt gaatatatcc tgccgccgct gccgctttgc acccggtgga 8460

gcttgcatgt tggtttctac gcagaactga gccggttagg cagataattt ccattgagaa 8520

ctgagccatg tgcaccttcc ccccaacacg gtgagcgacg gggcaacgga gtgatccaca 8580

tgggactttt 8590

<210> 2
<211> 7794
<212> DNA
<213> Artificial

<220>
<223> recombinant DNA construct in plasmid between Agrobacterium
      borders

<400> 2
aggatttttc ggcgctgcgc tacgtccgcg accgcgttga gggatcaagc cacagcagcc 60

cactcgacct tctagccgac ccagacgagc caagggatct ttttggaatg ctgctccgtc 120

gtcaggcttt ccgacgtttg ggtggttgaa cagaagtcat tatcgcacgg aatgccaagc 180

actcccgagg ggaaccctgt ggttggcatg cacatacaaa tggacgaacg gataaacctt 240

ttcacgccct tttaaatatc cgattattct aataaacgct cttttctctt aggtttaccc 300

gccaatatat cctgtcaaac actgatagtt taaactgaag gcgggaaacg acaatctgat 360

ccccatcaag cttactcgag gtcattcata tgcttgagaa gagagtcggg atagtccaaa 420

ataaaacaaa ggtaagatta cctggtcaaa agtgaaaaca tcagttaaaa ggtggtataa 480

agtaaaatat cggtaataaa aggtggccca aagtgaaatt tactcttttc tactattata 540

aaaattgagg atgtttttgt cggtactttg atacgtcatt tttgtatgaa ttggttttta 600

agtttattcg cttttggaaa tgcatatctg tatttgagtc gggttttaag ttcgtttgct 660

tttgtaaata cagagggatt tgtataagaa atatctttag aaaaacccat atgctaattt 720

gacataattt ttgagaaaaa tatatattca ggcgaattct cacaatgaac aataataaga 780

ttaaaatagc tttcccccgt tgcagcgcat gggtattttt tctagtaaaa ataaaagata 840

aacttagact caaaacattt acaaaaacaa cccctaaagt tcctaaagcc caaagtgcta 900

tccacgatcc atagcaagcc cagcccaacc caacccaacc caacccaccc cagtccagcc 960

aactggacaa tagtctccac acccccccac tatcaccgtg agttgtccgc acgcaccgca 1020

cgtctcgcag ccaaaaaaaa aaagaaagaa aaaaaagaaa aagaaaaaac agcaggtggg 1080

tccgggtcgt gggggccgga aacgcgagga ggatcgcgag ccagcgacga ggccggccct 1140

ccctccgctt ccaaagaaac gccccccatc gccactatat acataccccc ccctctcctc 1200

ccatcccccc aaccctacca ccaccaccac caccacctcc acctcctccc ccctcgctgc 1260

cggacgacga gctcctcccc cctccccctc cgccgccgcc gcgccggtaa ccaccccgcc 1320
cctctcctct ttctttctcc gttttttttt ccgtctcggt ctcgatcttt ggccttggta 1380
gtttgggtgg gcgagaggcg gcttcgtgcg cgcccagatc ggtgcgcggg aggggcggga 1440
tctcgcggct ggggctctcg ccggcgtgga tccggcccgg atctcgcggg gaatggggct 1500
ctcggatgta gatctgcgat ccgccgttgt tgggggagat gatggggggt ttaaaatttc 1560
cgccgtgcta aacaagatca ggaagagggg aaaagggcac tatggtttat atttttatat 1620
atttctgctg cttcgtcagg cttagatgtg ctagatcttt ctttcttctt tttgtgggta 1680
gaatttgaat ccctcagcat tgttcatcgg tagtttttct tttcatgatt tgtgacaaat 1740
gcagcctcgt gcggagcttt tttgtaggta gaagtgatca accatggcgc aagttagcag 1800
aatctgcaat ggtgtgcaga acccatctct tatctccaat ctctcgaaat ccagtcaacg 1860
caaatctccc ttatcggttt ctctgaagac gcagcagcat ccacgagctt atccgatttc 1920
gtcgtcgtgg ggattgaaga agagtgggat gacgttaatt ggctctgagc ttcgtcctct 1980
taaggtcatg tcttctgttt ccacggcgtg catgcttcac ggtgcaagca gccggcccgc 2040
aaccgcccgc aaatcctctg gcctttccgg aaccgtccgc attcccggcg acaagtcgat 2100
ctcccaccgg tccttcatgt tcggcggtct cgcgagcggt gaaacgcgca tcaccggcct 2160
tctggaaggc gaggacgtca tcaatacggg caaggccatg caggcgatgg gcgcccgcat 2220
ccgtaaggaa ggcgacacct ggatcatcga tggcgtcggc aatggcggcc tcctggcgcc 2280
tgaggcgccg ctcgatttcg gcaatgccgc cacgggctgc cgcctgacga tgggcctcgt 2340
cggggtctac gatttcgaca gcaccttcat cggcgacgcc tcgctcacaa agcgcccgat 2400
gggccgcgtg ttgaacccgc tgcgcgaaat gggcgtgcag gtgaaatcgg aagacggtga 2460
ccgtcttccc gttaccttgc gcgggccgaa gacgccgacg ccgatcacct accgcgtgcc 2520
gatggcctcc gcacaggtga agtccgccgt gctgctcgcc ggcctcaaca cgcccggcat 2580
cacgacggtc atcgagccga tcatgacgcg cgatcatacg gaaaagatgc tgcagggctt 2640
tggcgccaac cttaccgtcg agacggatgc ggacggcgtg cgcaccatcc gcctggaagg 2700
ccgcggcaag ctcaccggcc aagtcatcga cgtgccgggc gacccgtcct cgacggcctt 2760
cccgctggtt gcggccctgc ttgttccggg ctccgacgtc accatcctca acgtgctgat 2820
gaaccccacc cgcaccggcc tcatcctgac gctgcaggaa atgggcgccg acatcgaagt 2880
catcaacccg cgccttgccg gcggcgaaga cgtggcggac ctgcgcgttc gctcctccac 2940
gctgaagggc gtcacggtgc cggaagaccg cgcgccttcg atgatcgacg aatatccgat 3000
tctcgctgtc gccgccgcct tcgcggaagg ggcgaccgtg atgaacggtc tggaagaact 3060
ccgcgtcaag gaaagcgacc gcctctcggc cgtcgccaat ggcctcaagc tcaatggcgt 3120
ggattgcgat gagggcgaga cgtcgctcgt cgtgcgtggc cgccctgacg gcaaggggct 3180

cggcaacgcc tcgggcgccg ccgtcgccac ccatctcgat caccgcatcg ccatgagctt 3240

cctcgtcatg ggcctcgtgt cggaaaaccc tgtcacggtg gacgatgcca cgatgatcgc 3300

cacgagcttc ccggagttca tggacctgat ggccgggctg ggcgcgaaga tcgaactctc 3360

cgatacgaag gctgcctgat gagctcgaat tcccgatcgt tcaaacattt ggcaataaag 3420

tttcttaaga ttgaatcctg ttgccggtct tgcgatgatt atcatataat ttctgttgaa 3480

ttacgttaag catgtaataa ttaacatgta atgcatgacg ttatttatga gatgggtttt 3540

tatgattaga gtcccgcaat tatacattta atacgcgata gaaaacaaaa tatagcgcgc 3600

aaactaggat aaattatcgc gcgcggtgtc atctatgtta ctagatcggg gatgggggat 3660

ccactagtga tatccgtcga ctggtaccta cgcgtagcta gcccgggcgc gccttaatta 3720

agcggccgct tcgagtggct gcaggtcgat tgatgcatgt tgtcaatcaa ttggcaagtc 3780

ataaaatgca ttaaaaaata ttttcatact caactacaaa tccatgagta taactataat 3840

tataaagcaa tgattagaat ctgacaagga ttctggaaaa ttacataaag gaaagttcat 3900

aaatgtctaa aacacaagag gacatacttg tattcagtaa catttgcagc ttttctaggt 3960

ctgaaaatat atttgttgcc tagtgaataa gcataatggt acaactacaa gtgttttact 4020

cctcatatta acttcggtca ttagaggcca cgatttgaca catttttact caaaacaaaa 4080

tgtttgcata tctcttataa tttcaaattc aacacacaac aaataagaga aaaaacaaat 4140

aatattaatt tgagaatgaa caaaaggacc atatcattca ttaactcttc tccatccatt 4200

tccatttcac agttcgatag cgaaaaccga ataaaaaaca cagtaaatta caagcacaac 4260

aaatggtaca agaaaaacag ttttcccaat gccataatac tcaaactcag taggattctg 4320

gtgtgtgcgc aatgaaactg atgcattgaa cttgacgaac gttgtcgaaa ccgatgatac 4380

gaacgaaagc tgaattccta gctggctgaa tggtagtagt tgttgctgct gtaaataagc 4440

aggagagttc aatgctgtca gttggttgaa tggaagaaat tgctgggggt aggcagcaga 4500

tagctggctg aatggtagtt gttgttgttg caaataagaa gcagagttca atgcagctag 4560

ttggttgaat ggaagaaact gctgttgctg agagtaggca gcaaggtttg ctagcacaag 4620

ttgttgtagt tgttgtgccc tgatgttttg tgccaataaa tgcaccaaag gtaactgctg 4680

taatagggct gatgattgtt ggaggaacaa gggtgataaa ggtaagatgc cagctgcgat 4740

tgcctgttat gcataaagat ggcacctcca acgatgggtt gctgcaaggc agggttcatc 4800

aaagagaact ggttgtatgg cagcaattgt tgttgctgct gcaggaaggt agcgaccaat 4860

gggttagcca ctgccaatgg attaagtaac tgttgtcgct gttgtaggta cgcagcagag 4920

tttgacacag ccagttggtt gaatggaagc aactgttgta agtaggcagc agggtttgcc 4980

acagctagct gagtcagagc tggtacaatt tgttgcagca actgttgttg taggtacgta 5040

ggtgggcccg ctaccaagat attagccctc cttgcgcttc ttgccctttt agtgagcgca 5100

acaaatgcgt tcattattcc acagtgctca cttgctccta gtgccagtat tccacagttc 5160

ctcccaccag ttacttcaat gggcttcgaa catccagccg tgcaagccta caggctacaa 5220

ctagcgcttg cggcgagcgc cttacaacaa ccaattgccc aattgcaaca acaatccttg 5280

gcacatctaa ccctacaaac cattgcaacg caacaacaac aacaacagtt tctgccatca 5340

ctgagccacc tagccgtggt gaaccctgtc acctacttgc aacagcagct gcttgcatcc 5400

aacccacttg ctctggcgaa cgtagctgca taccagcaac aacaacagct gcaacagttt 5460

atgccagtgc tcagtcaact agccatggtg aaccctgccg tctacctaca actactttca 5520

tctagcccgc tcgcggtggg caatgcacct acgtacctac aacaacagtt gctgcaacaa 5580

attgtaccag ctctgactca gctagctgtg gcaaaccctg ctgcctactt acaacagttg 5640

cttccattca accaactggc tgtgtcaaac tctgctgcgt acctacaaca gcgacaacag 5700

ttacttaatc cattggcagt ggctaaccca ttggtcgcta ccttcctgca gcagcaacaa 5760

caattgctgc catacaacca gttctctttg atgaaccctg ccttgcagca acccatcgtt 5820

ggaggtgcca tctttaccgg taacaggcaa tcgcagctgg catcttacct ttatcaccct 5880

tgttcctcca acaatcatca gccctattac agcagttacc tttggtgcat ttattggcac 5940

aaaacatcag ggcacaacaa ctacaacaac ttgtgctagc aaaccttgct gcctactctc 6000

agcaacaaca gtttcttcca ttcaaccaac tagctgcatt gaactctgct tcttatttgc 6060

aacaacaaca actaccattc agccagctat ctgctgccta cccccagcaa tttcttccat 6120

tcaaccaact gacagctttg aactctcctg cttatttaca gcagcaacaa ctactaccat 6180

tcagccagct agggatccgg taccgggttc ttctgcgctc tggagtagat aaagctaatg 6240

gtctgaagac ccagtggtgg tgatggagaa gtgcacaggc atgcgagcgt tatttatagc 6300

tttgattaat taacacaatt tcttgtgttc ttatgccacc gagacggctg taggcagctt 6360

catggtttct tgccaaatgt atatgactcg tcactctctt tacgtagcac gtcgatggtt 6420

catctggaat cattctgtac ttctgcgtgg ctcagttttg ttgccttcta caggttgttg 6480

atctacgtaa aacgaattag atttagcttg acatatggct ttttttttgt tgtaaattta 6540

ctttacacgt caaggatttt tgtcctgtcc ggcctatttt atttttcatg aaacgatctt 6600

tgtaatgcaa tatgagttgt ttgtaatgtc ttgtgagctg taagcatgta tatcagatga 6660

gtatgatctc ggcatgactc accgtgtttc tttgcacaca gagaggattt gtttgattgt 6720

ttcttaccca atacccttga cgtgcaattt tggttgatgt tctgtgagtt gttaaggata 6780

caacaaattc ttggagcttt acatgccaat gcatggttgt ttcgtgttcc tcaccacttt 6840

aggacttata cggttgcacc tggatgatcg aaggggattg ggagagatta aatctccttc 6900

tattcaattt tgactaggaa gagatttaat cgtttccaac ccctttcgat ccagacgtaa 6960

gcgaacaagt tttttatttg gataccctct tattcatctt aatacacaca tgtattaagt 7020

tgcactagtt atatgcccgt gcattgctac ggtttatata tatatatata tatatgtata 7080

tatatatata tgatatatga taaattttgt tttaataaaa catatgtttt ctattgatta 7140

ggttgtgtga atatggagcc aacaaccaat atccagaaca cttatacata atttcacctt 7200

attttgtaca taaactctct tattatagta gtagagaaga gattataaga gtgcgggttg 7260

attataaaga aatgtaggag ttttttaata atattgacgc gggacaagct tactagtagc 7320

ttgttaacgc ggccgcatcg atcgtgaagt ttctcatcta agcccccatt tggacgtgaa 7380

tgtagacacg tcgaaataaa gatttccgaa ttagaataat ttgtttattg ctttcgccta 7440

taaatacgac ggatcgtaat ttgtcgtttt atcaaaatgt actttcattt tataataacg 7500

ctgcggacat ctacattttt gaattgaaaa aaaattggta attactcttt ctttttctcc 7560

atattgacca tcatactcat tgctgatcca tgtagatttc ccggacatga agccatttac 7620

aattgaatat atcctgccgc cgctgccgct ttgcacccgg tggagcttgc atgttggttt 7680

ctacgcagaa ctgagccggt taggcagata atttccattg agaactgagc catgtgcacc 7740

ttccccccaa cacggtgagc gacggggcaa cggagtgatc cacatgggac tttt 7794